# EUROPEAN PATENT SPECIFICATION

(11) **EP 2 631 629 B1**
(45) Date of publication and mention of the grant of the patent: **09.03.2016**
(21) Application number: 12849242.8
(22) Date of filing: 17.12.2012
(51) Int. Cl.: G01N 1/28, B09B 3/00

(54) **TEST PIECE MANUFACTURING SYSTEM AND METHOD**
HERSTELLUNGSSYSTEM UND -VERFAHREN FÜR TESTSTÜCKE
SYSTÈME ET PROCÉDÉ DE FABRICATION D'ÉPROUVETTE

(30) Priority: 14.09.2012 JP 2012203300
(43) Date of publication of application: 28.08.2013
(73) Proprietor: Fuji Corporation Co., Ltd., Saku-shi, Nagano 385-0009 (JP)
(72) Inventor: YAMAGUCHI, Tokichiro, Saku-shi Nagano 385-0009 (JP)
(74) Representative: Hofmann, Ralf U.
(86) International application number: PCT/JP2012/082688
(87) International publication number: WO 2013/073716

(56) References cited:
- JP-A- 2005 017 171
- JP-A- 2008 200 586
- JP-A- 2011 117 847
- Nukem Gmbh: "CEMENTATION OF RADIOACTIVE WASTE", , 30 June 2007 (2007-06-30), XP055127793, Hanau Retrieved from the Internet: URL:http://www.nukemgroup.com/fileadmin/pd f/Brochure_Cementation_Juni_2007.pdf [retrieved on 2014-07-09]

## Description

### Technical Field

The present invention relates to a system and method for producing a test piece that reproduces a state in which a banking material that contains inorganic waste is landfilled.

### Background Art

Conventionally, as this type of test piece, a method for producing a test piece of CSG (Cement Sand and Gravel) material is known in which CSG material is produced by adding water and an additive to cement and waste such as asbestos and mixing the water, additive cement and waste, and the produced CSG material is filled into a molding form to be molded, and compressive strength and the like of the molded object is evaluated, as described in the undermentioned Patent Literature 1 and Patent Literature 2.

### Prior Art Literature

### Patent Literature

Patent Literature 1: Unexamined Japanese Patent Application Kokai Publication No. 2008-200586
Patent Literature 2: Unexamined Japanese Patent Application Kokai Publication No. 2005-17171

### Disclosure of the Invention

### Problem to be Solved by the Invention

However, such a conventional test piece is only for evaluating CSG that contains cement, and it is difficult to reproduce a state in which inorganic waste is landfilled at a final disposal site.

The present invention has an objective to provide a system and method for producing a test piece that can reproduce a state in which a banking material that contains inorganic waste is landfilled at a final disposal site.

### Means for Solving the Invention

A system for producing a test piece according to a first aspect of the present invention produces a test piece that reproduces a state in which a banking material that contains inorganic waste is landfilled, and includes:
mixers configured to produce the banking material and a covering material that covers the banking material, respectively, by mixing;
a flat-bottomed, cylindrical molding form that has a removable lid;
an air-press device having a support base to which the molding form can be fixed, and is configured to shock-compress, at each step, the covering material and the banking material poured into the molding form in a stepwise fashion;
an air compressor configured to operate the air-press device; and
a storage space configured to store the ancillary equipment with the mixer, the molding form and the air-press device;
the mixers and the air compressor being disposed in a right and left direction at each side of and adjacent to the air-press device; and the storage space being disposed in a front and back direction to the air-press device with work space between the storage space and the air-press device.

In the system for producing a test piece according to the first aspect of the present invention, the mixers produce, by mixing, the banking material and the covering material, respectively, both being materials of the test piece. The produced banking material and covering material are poured into the molding form. Since the molding form is fixed to the support base of the air-press device, materials can be poured using the support base of the air-press device as a working table.

Into the molding form, the covering material, banking material and covering material are poured in this order in a stepwise fashion, and each time the material is poured, the material is shock-compressed by the air-press device. By shock compressing, the state in which the respective materials are casted at a final disposal site can be reproduced.

Since the mixers and the air compressor are disposed in a right and left direction at each side of and adjacent to the air-press device, the materials produced by the mixers can be easily poured into the molding form of the air-press device adjacent to the mixers. Furthermore, a series of actions to operate the air compressor to supply operating air to the air-press device can be easily performed since the air compressor and air-press device are adjacent to each other, thereby enabling the air-press device to efficiently shock-compress the materials poured into the molding form.

In addition, since the storage space for accommodating ancillary equipment that is used with the mixers, the molding form and the air-press device is disposed in a front and back direction to the air-press device with work space between the storage space and the air-press device, a worker can pour the materials from the mixers into the molding form and operate the air compressor in the same workflow line, as well as can easily take out and store the ancillary equipment that is used during these works.

In this way, the system for producing a test piece according to the first aspect of the present invention can easily reproduce a state in which the banking material that contains inorganic waste is landfilled at the final disposal site.

The inorganic waste includes toxic substance, radioactive substance, and mixtures thereof, in addition to incinerated ash of general waste such as garbage and waste resin.

In a system for producing a test piece according to a second aspect of the present invention, the system for producing a test piece according to the first aspect is disposed on the bed of a freight automobile in such a way that the right and left direction of the system for producing corresponds to the front and back direction of the freight automobile.

In the system for producing a test piece according to the second aspect, since the system is installed on the bed of the freight automobile, the state in which a banking material that contains inorganic waste is landfilled at a final disposal site can be easily reproduced at any place.

Where the system is installed on the bed of the freight automobile, the workflow line may be obstructed due to limitations of space of the bed. However, by making the right and left direction of the system correspond to the front and back direction, which is a longer direction of the bed of the freight automobile, work space for the workflow line can be secured.

In this way, with the system for producing a test piece according to the second aspect of the invention, the state in which a banking material that contains inorganic waste is landfilled at a final disposal site can be easily reproduced at any place.

A method for producing a test piece according to a third aspect of the present invention produces a test piece that reproduces a state in which a banking material that contains inorganic waste is landfilled, and includes:
a first step of pouring a covering material onto the bottom of a flat-bottomed cylindrical molding form, and compressing the covering material to be compacted, thereby reproducing a lower capping layer of the compacted covering material;
a second step of pouring the banking material onto the covering material reproducing the lower capping layer in the first step, and compressing the banking material, thereby reproducing the banking material in a consolidation state;
a third step of pouring the covering material onto the banking material in a consolidation state reproduced with the second step, and compressing the covering material to be compacted, thereby reproducing an upper capping layer of the compacted covering material; and
a fourth step of fixing the top of the covering material reproducing the upper capping layer in the third step by a lid.

In the method for producing a test piece according to the third aspect of the present invention, at the first step, the covering material is first poured onto the bottom of the molding form, and compressed, thereby reproducing a lower capping layer disposed under the banking material, of capping layers that shield the banking material that contains inorganic waste at a final disposal site.

Next, at the second step, the same banking material as a banking material to be actually landfilled at the final disposal site is placed onto the covering material that has reproduced the lower capping layer, and is compressed, thereby reproducing the banking material in a consolidation state at the final disposal site.

Next, at a third step, the covering material is poured onto the banking material in a consolidation state reproduced, and is compressed, thereby reproducing an upper capping layer disposed on the top of the banking material, of the capping layers that shield the banking material at the final disposal site.

Next, at a fourth step, the top of the covering material that has reproduced the upper capping layer is fixed with a lid, thereby reproducing a state in which a weight for suppressing expansion is placed at the final disposal site.

The test piece produced in this way reproduces a state in which the banking material that contains inorganic waste is landfilled at the final disposal site. For example, without a lid, the state of the ground surface of the final disposal site can be reproduced.

In this way, the method for producing a test piece according to the third aspect of the present invention can reproduce a state in which the banking material that contains inorganic waste is landfilled at the final disposal site.

A method for producing a test piece according to a fourth aspect of the present invention includes a pre-processing step of disposing a resin material for exfoliation on the inside surface of the molding form, prior to the first step in the third aspect.

In the method for producing a test piece according to the fourth aspect of the present invention, since the resin material for exfoliation is disposed on the inside surface of the molding form at the pre-processing step, after producing the test piece, the test piece can be easily separated from the molding form (for performing a dissolution test of the test piece or for discarding the test piece).

In this way, the method for producing a test piece according to the fourth aspect of the present invention can make it easy to handle the test piece, and also reproduce a state in which the banking material that contains inorganic waste is landfilled at the final disposal site.

A method for a test piece according to a fifth aspect of the present invention includes a post-processing step of curing the test piece for a certain period while the covering material reproducing the upper capping layer in the fourth step is fixed with the lid, and then removing the lid and molding form.

In the method for producing a test piece according to the fifth aspect of the present invention, the post-processing step can reproduce a state in which the banking material that contains inorganic waste that have been cured for a certain period is landfilled at the final disposal site. Therefore, after the post-processing step, the test piece removed from the lid and molding form can be used for a dissolution test and the like.

In this way, the method for producing the test piece according to the fifth aspect of the present invention can reproduce a state in which the banking material that contains inorganic waste is landfilled at the final disposal site, thereby allowing for evaluation of the effects of landfilling the inorganic waste at the final disposal site.

### Brief Description of Drawings

FIG. 1 is a top view illustrating a system for producing a test piece according to the present embodiment;
FIG. 2 is an illustration showing a configuration of the system for producing the test piece in FIG. 1;
FIG. 3 is an exploded perspective view illustrating members used for a method for producing the test piece according to the present embodiment;
FIG. 4 is a flow chart illustrating steps of the method for producing the test piece according to the present embodiment;
FIG. 5 is a perspective view illustrating an assembled state of members in FIG. 3;
FIG. 6 is a cross-sectional view taken on line VI-VI of FIG. 5; and
FIG. 7 is an illustration for showing production of the test piece using the members in FIG. 3.

### Mode for Carrying out the Invention

A system for producing a test piece according to one embodiment of the present invention will be described.

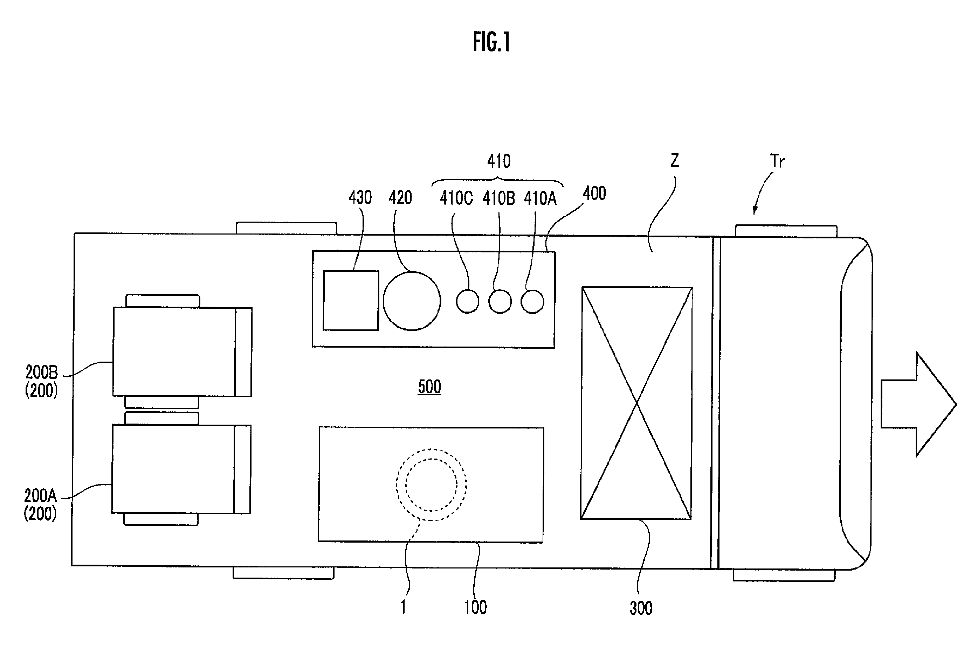

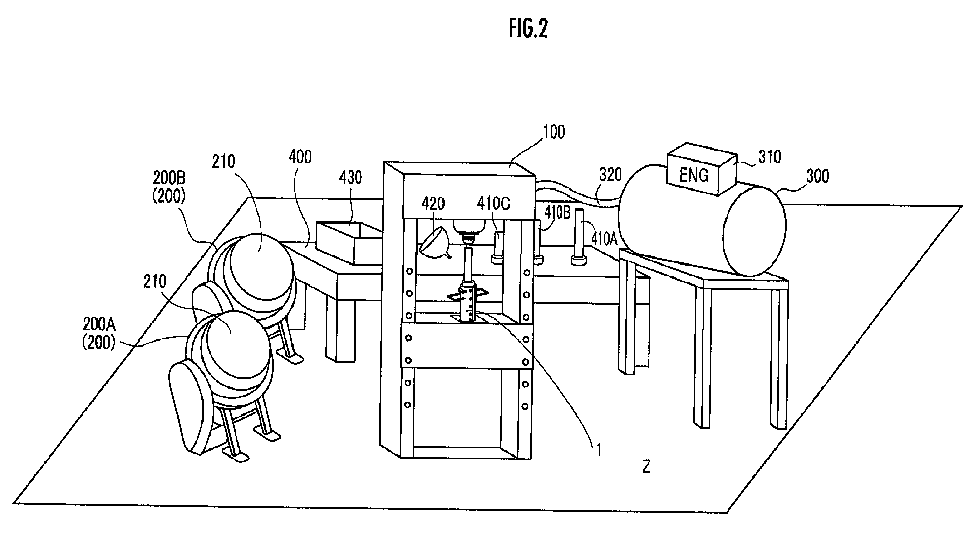

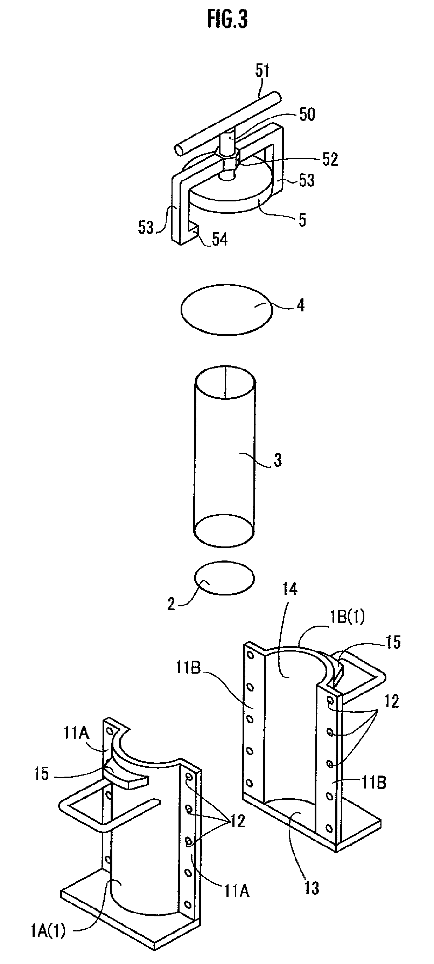

As illustrated in FIGS. 1 and 2, the system for producing the test piece is installed on a bed Z of a truck Tr that is a freight automobile, and includes: a molding form 1 (that will be described later with reference to FIG. 3) into which materials are poured; an air-press device 100 configured to shock-compress the materials poured into the molding form 1; mixers 200 configured to produce the materials to be poured into the molding form 1 by mixing; an air compressor 300 (including a driving engine 310) configured to operate the air-press device 100; and a storage space 400 to store compression members 410, a funnel 420 and a material storage container 430 that are ancillary equipment used with the above.

The mixers 200 and the air compressor 300 are disposed in a right and left direction at each side of and adjacent to the air-press device 100. Further, the storage space 400 is disposed in a front and back direction to the air-press device 100 with work space 500 between the storage device 400 and the air-press device 100.

This is a whole configuration of the system for producing a test piece according to the present embodiment.

Next, details of each component will be described.

As illustrated in FIG. 3, in order to produce a test piece, molding form members including the molding form 1, a bottom surface resin material 2, a side surface resin material 3, a top surface resin material 4 and a lid 5 are used.

The molding form 1 is of a flat-bottomed cylindrical shape, and composed of two steel semi-cylinders 1A, 1B connected to each other. The two semi-cylinders 1A, 1B have the same length, inner diameter, and wall thickness (length 300 mm; inner diameter 115.8 mm; wall thickness 12 mm), and edges of both ends of the two semi-cylinders 1A, 1B are connected to each other so that the two semi-cylinders 1A, 1B compose a cylinder.

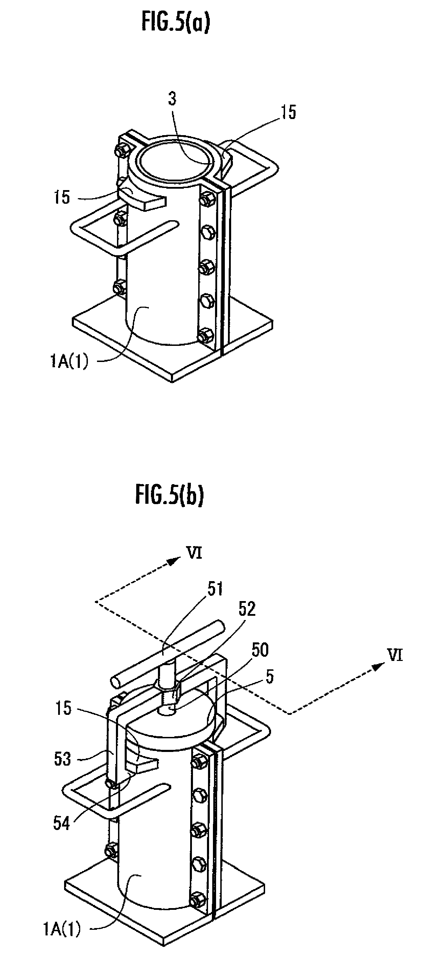

More specifically, the two semi-cylinders 1A, 1B are connected in such a way that flanges 11A, 11A formed at both ends of the semi-cylinder 1A face flanges 11B, 11B formed at both ends of the semi-cylinder 1B, respectively, and bolts and nuts are used for through-holes 12 formed in the flanges 11A, 11A and flanges 11B, 11B (see FIG. 5).

The bottom surface resin material 2, the side surface resin material 3, he top surface resin material 4 are disposed on inner surfaces of the molding form I and the lid 5 for exfoliation. In the present embodiment, polycarbonate resin is used.

That is, the bottom surface resin material 2 is a circular film that matches the shape of the bottom surface 13 of the molding form 1, the side surface resin material 3 is a cylindrical film formed by rolling a rectangular film of developed shape of an inner side surface 14 of the molding form 1, and the top surface resin material 4 is a circular film that matches the shape of the bottom surface of the lid 5.

The lid 5 has a disk-like shape for closing the top of the molding form 1, and has a slightly larger diameter than the outer diameter of the molding form 1.

The lid 5 has a torsion bar 50 that extends upward from the center thereof. The torsion bar 50 has a male thread groove (not illustrated) formed at the side surface thereof. To the upper end of the torsion bar 50 is connected a handle 51 that extends horizontally rightward and leftward.

The lid 5 has a pair of support members 53, 53 that are bent so as to pinch a nut 52 therein, the nut 52 screwing with the male thread groove of the torsion bar 50. Hooks 54, 54 at the tips of the support members 53, 53 can engage with the engagements 15, 15 provided at the outer surface of the molding form 1.

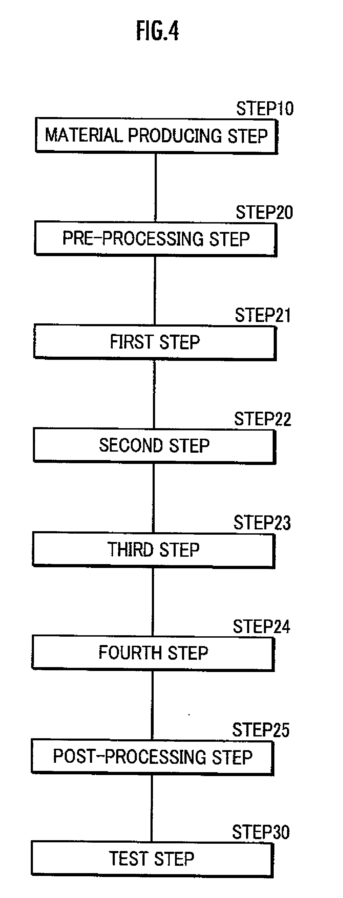

Next, with reference to FIGS. 4 to 6, a method for producing a test piece according to the present embodiment will be described.

As illustrated in FIG. 2, first, materials such as a covering material and a banking material are produced (STEP 10: material producing step in FIG. 4).

At this material producing step, a mixer 200A is used for producing a covering material, which produces cement mixes as a covering material by mixing cement with sand and water; and a mixer 200B is used for producing a banking material, which produces the banking material for a test by mixing inorganic waste with cement and water.

In specific works in the material producing step, cement and so on are poured into each of the mixers 200A and 200B with the lid 210 open and mixed for a certain time with the lid 210 closed, and then produced materials are taken out of the mixers with the lid 210 open. The taken-out materials are transferred to the material storage container 430.

Here, inorganic waste is, for example, incinerated ash to be landfilled at a final disposal site, a banking material is classified into Type-A banking material whose toxic substance concentration is a predetermined reference value or below, and Type-B banking material whose toxic substance concentration is over the predetermined reference value, according to a type of incinerated ash.

Toxic substances include toxic substance (dioxin) contained in incinerated ash of general waste such as garbage and waste plastic, and heavy metal as well as radioactive substance. Depending on whether the concentration of any one of these toxic substances exceeds a reference value (for example, 100Bq/kg for a radioactive amount of radioactive substance), inorganic waste is classified into Type-A inorganic waste and Type-B inorganic waste.

In the present embodiment, Type-A inorganic waste is a waste in which various toxic substance concentrations are the respective predetermined reference values or below. However, Type-A inorganic waste may be a waste whose toxic substance concentration is lower than those of Type-B inorganic waste (regardless of a reference value).

Next, a worker connects the two semi-cylinders 1A, 1B with bolts and nuts to assemble the molding form 1, and then disposes a resin material for exfoliation on the inner surface of the molding form 1 (STEP20: pre-processing step in FIG. 4).

At this pre-processing step, the bottom surface resin material 2 is placed on the bottom surface 13 of the molding form 1, and the side surface resin material 3 is rolled into a cylinder shape and placed on the inner side surface 14 of the molding form 1. This state is illustrated in FIG. 5A.

In the state illustrated in FIG. 5A, the worker pours a covering material into the molding form 1, and compresses the covering material (STEP21: first step in FIG. 4).

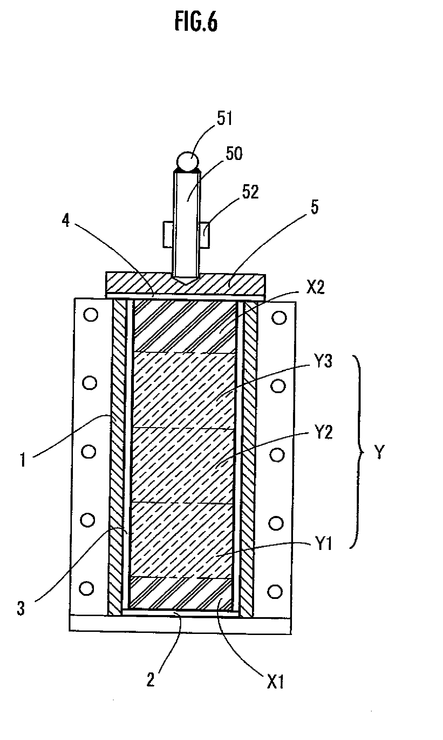

At the first step, as schematically illustrated in FIG. 6, a covering material X1 is poured onto the bottom of the molding form 1, and shock-compressed, thereby reproducing a lower capping layer made of the compacted covering material X1.

The covering material X1 is poured from the material container 430 into the molding form 1 without leaking with the use of the funnel 420 (hereinafter, materials will be similarly poured into the molding form 1 from the material storage container 430 with the use of the funnel 420).

To compress the covering material X1, the air-press device 100 is used. At least first to fourth steps are performed in the state where the molding form 1 is fixed to a support base 110 of the air-press device 100.

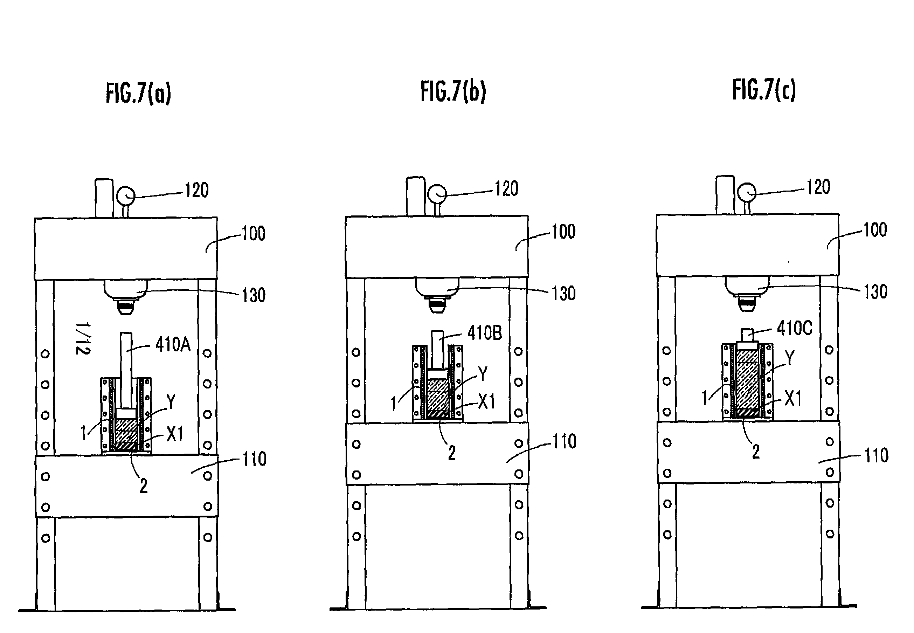

In the air-press device 100, a pressure portion 130 moves up and down according to the up and down movement of the handle 120 that is caused by compressed air supplied via a supply tube 320 from the air compressor 300. At this time, since each stroke of the pressure portion 130 is limited, a long-stroke compression member 410A of piston-like compression members 410 is used, as illustrated in FIG. 7A, in order to press the covering material X1 on the bottom of the molding form 1. The compression members 410A to 410C have the same piston portions on the tips thereof (outer diameter: 106 mm), and have different stroke lengths.

The air-press device 100 makes the working speed of the pressure portion 130 faster, thereby pressurizing with impact by striking strokes, unlike a case of a continuous pressurizing. This permits pressurizing the covering material X1a number of times with a pressure of 25 tons per time, thereby reproducing a state in which the covering material X1 is casted as the lower capping layer at a final disposal site.

The reproduced lower capping layer corresponds to an upper capping layer of the next layer stack below at an actual final disposal site, since a plurality of layer stacks are formed there.

Next, the worker places the banking material preliminarily produced at STEP 10 on the covering material X1 that has reproduced the lower capping layer and compresses the banking material (STEP22: second step in FIG. 4).

At this second step, as illustrated in FIG. 6, a banking material Y1 is placed onto the covering material X1 that has reproduced the lower capping layer and compressed in the molding form 1, thereby reproducing the banking material Y1 in a consolidation state at the final disposal site.

A banking material Y is consolidation-molded to a plurality of layers by repeatedly pouring the banking material Y into the molding form 1 and using the air-press device 100 to shock-press the banking material Y as illustrated in FIGS. 7A to 7C in this order. At this time, the long-stroke compression member 410A to short-stroke compression members 410B and 410C are properly selected and used from the compression members 410 according to the lamination state (a lamination height) of the banking material Y, as illustrated in FIGS 7A to 7C in this order.

The same banking material Y may be used to reproduce consolidation-molded layers, or part of banking materials Y1, Y2, Y3 in FIG. 6 may be a different banking material according to a disposal state at the final disposal site.

For example, the banking materials Y1 and Y2 may be Type-B banking material whose toxic substance concentration is over a predetermined reference value and the banking material Y3 may be Type-A banking material whose toxic substance concentration is the predetermined reference value or below. This enables the test piece to reflect the disposal state at the final disposal site where Type-A banking material covers Type-B banking material.

Next, the worker pours a covering material X2 onto the banking material Y in a consolidation state and compresses the covering material X2 (STEP23: third step in FIG. 4).

At this third step, as schematically illustrated in FIG. 6, the same covering material X2 as the covering material X1 is poured onto the banking material Y in a consolidation state and compressed, thereby reproducing an upper capping layer that shields the banking material Y at the final disposal site. Also at this step, the air-press device 100 is used to compress the covering material X2.

Next, the worker puts the top surface resin material 4 on the covering material X2 that has reproduced the upper capping layer and fixes the lid 5 (STEP24: fourth step in FIG. 4).

Specifically, as illustrated in FIG. 5B, the lid 5 is placed on the molding form 1 with the hooks 54, 54 of the pair of support members 53, 53 engaging with the engagements 15, 15 of the molding form 1. In this state, by turning the handle 51, the lid 5 connected to the torsion bar 50 moves downward relative to the pair of support members 53, 53 to be fixed to the molding form 1.

As illustrated in a cross-sectional view in FIG. 6, by fixing the top of the covering material X2 that has reproduced the upper capping layer with the lid 5, the lid 5 can pressure-fix the covering material X2 even if the banking materials Y1 to Y3 expand. Therefore, according to the fourth step, a state in which a weight for suppressing expansion is placed at the final disposal site is reproduced.

Next, after curing the test piece for a certain period with the lid 5 being fixed on the molding form 1, the worker removes the lid 5 and molding form 1 (STEP25: post-processing step in FIG. 4).

At this time, since the bottom surface resin material 2, the side surface resin material 3 and the top surface resin material 4 exist between the molding form 1 and lid 5 and the produced test piece, the molding form 1 and lid 5 can be easily removed without the test piece adhering to the molding form 1 and lid 5.

At this post-processing step, by curing the test piece for a certain period with the lid 5 being fixed on the molding form 1, a state in which curing is performed for a certain period with a weight for suppressing expansion being placed at the final disposal site can be realized. Therefore, a state in which a banking material that contains inorganic waste is landfilled at the final disposal site may be reproduced.

The above is a method for producing a test piece according to the present embodiment, which can reproduce a banking material Y that contains inorganic waste is landfilled at the final disposal site.

Therefore, the worker may use the test piece removed from the lid 5 and molding form 1 after the post-processing step in STEP 25 to perform various tests such as a dissolution test and evaluation (STEP 30 in FIG. 4). This enables the effect of landfilling the inorganic waste at the final disposal site to be preliminarily evaluated.

The test piece produced in the method for producing a test piece according to the present embodiment is actually tested and evaluated to result in the uniaxial compressive strength being 22.09 [MN/m²], which is the same as the uniaxial compressive strength of the banking material Y that contains inorganic waste that is landfilled at a final disposal site. This shows that the test piece has reproduced a state in which the banking material Y that contains inorganic waste is landfilled at the final disposal site.

Furthermore, since the system for producing the test piece that performs a method for producing a test piece according to the present embodiment is installed on the bed of a truck that is a freight automobile, the state in which a banking material that contains inorganic waste is landfilled at a final disposal site can be easily reproduced at any place.

Furthermore, since the mixers 200 and the air compressor 300 are in a right and left direction (a front and back direction of the truck Tr) at each side and adjacent to the air-press device 100, materials mixed and produced by the mixers 200 can be easily poured into the molding form 1 of the air-press device 100. Furthermore, a series of actions to operate the air compressor 300 to supply operating air to the air-press device 100 can be easily performed at adjacent positions, enabling the air-press device 100 to efficiently shock-compress the materials poured into the molding form.

In addition, since the storage space 400 that accommodates ancillary equipment such as the compression members 410, the funnel 420 and the material storage container 430 is disposed in a front and back direction (a right and left direction of the truck Tr) to the air-press device 100 with work space 500 between the storage space 400 and the air-press device 100, a worker can pour materials from the mixers 200 to the molding form 1, and drive the air compressor 300 in the same workflow line. Furthermore, ancillary equipment used for these works can be easily taken out and stored.

In this way, the system for producing a test piece according to the present embodiment can improve workability and easily reproduce the state in which a banking material that contains inorganic waste is landfilled at a final disposal site.

In the present embodiment, the storage space 400 is a table for placing the compression members 410, the funnel 420 and the material storage container 430, but is not limited to the table, and may be a storage rack or a storage cabinet.

### Description of Reference Numerals

1 Molding form
1A, 1B Semi-cylinders
2 Bottom surface resin material
3 Side surface resin material
4 Top surface resin material
5. Lid
X1, X2 Covering material
Y1, Y2, Y3 Banking material
100 Air-press device
110 Support base
200A, 200B Mixer
300 Air compressor
400 Storage space
410A, 410B, 410C Compression members (ancillary equipment)
420 Funnel (ancillary equipment)
430 Material storage container (ancillary equipment)
500 Work space

## Claims

1. A system for producing a test piece that reproduces a state in which a banking material (Y1, Y2, Y3) that contains inorganic waste is landfilled,
comprising
mixers (200A, 200B) configured to produce the banking material (Y1, Y2, Y3) and a covering material (X1, X2) that covers the banking material (Y1, Y2, Y3), respectively, by mixing;
a flat-bottomed, cylindrical molding form (1),
**characterised in that** the system further comprises:
a removable lid (5) for for the form (1) configured for pressure-fixing the covering material (X1, X2) even if the banking material (Y1, Y2, Y3) expands;
an air-press device (100) having a support base (110) to which the molding form (1) can be fixed, and is configured to shock-compress, at each step, the covering material (X1, X2) and the banking material (Y1, Y2, Y3) poured into the molding form (1) in a stepwise fashion;
an air compressor (300) configured to operate the air-press device (100); and
a storage space (400) configured to store the ancillary equipment for the mixer (200A, 200B), for the molding form (1) and for the air-press device (100);
the mixers (200A, 200B) and the air compressor (300) being disposed in a right and left direction at each side of and adjacent to the air-press device (100); and the storage space (400) being disposed in a front and back direction to the air-press device (100) with work space (500) between the storage space (400) and the air-press device (100).

2. The system for producing a test piece according to claim 1, **characterized in that** the system is disposed on a bed of a freight automobile in such a way that the right and left direction of the production system corresponds to the front and back direction of the freight automobile.

3. A method for producing a test piece that reproduces a state in which a banking material (Y1, Y2, Y3) that contains inorganic waste is landfilled, **characterized by** comprising:
a first step of pouring a covering material (X1, X2) onto the bottom of a flat-bottomed cylindrical molding form (1), and compressing the covering material (X1, X2) to be compacted, thereby reproducing a lower capping layer of the compacted covering material (X1, X2);
a second step of pouring the banking material (Y1, Y2, Y3) onto the covering material (X1, X2) reproducing the lower capping layer in the first step, and compressing the banking material (Y1, Y2, Y3), thereby reproducing the banking material (Y1, Y2, Y3) in a consolidation state;
a third step of pouring the covering material (X1, X2) onto the banking material (Y1, Y2, Y3) in a consolidation state reproduced with the second step, and compressing the covering material (X1, X2) to be compacted, thereby reproducing an upper capping layer of the compacted covering material (X1, X2); and
a fourth step of fixing the top of the covering material (X1, X2) reproducing the upper capping layer in the third step by a lid (5), wherein the lid (5) pressure-fixes the covering material (X1, X2) even if the banking material (Y1, Y2, Y3) expands.

4. The method for producing a test piece according to claim 3, **characterized by** comprising pre-processing steps of disposing a resin material (2, 3, 4) for exfoliation on an inner surface of the molding form (1), prior to the first step.

5. The method for producing a test piece according to claim 3 or 4, **characterized by** comprising a post-processing step of curing the test piece for a certain period while the covering material (X1, X2) reproducing the upper capping layer in the fourth step is fixed with the lid (5), and then removing the lid (5) and molding form (1).

## Patentansprüche

1. System zur Herstellung eines Prüflings, der einen Zustand reproduziert, in welchem ein Aufschüttmaterial (Y1, Y2, Y3), das anorganischen Abfall enthält, deponiert wird, mit
Mischern (200A, 200B), die dafür konfiguriert sind, das Aufschüttmaterial (Y1, Y2, Y3) bzw. ein Abdeckmaterial (X1, X2), welches das Aufschüttmaterial (Y1, Y2, Y3) abdeckt, durch Mischen herzustellen,
einer zylindrischen Pressform (1) mit flachem Boden,
**dadurch gekennzeichnet, dass** das System ferner umfasst:
einen abnehmbaren Deckel (5) für die Form (1), der dafür konfiguriert ist, das Abdeckmaterial (X1, X2), selbst dann, wenn sich das Schüttmaterial (Y1, Y2, Y3) ausdehnt, unter Druck zu fixieren,
eine Luftdruckpressvorrichtung (100), die einen Auflagesockel (110) aufweist, auf dem die zylindrische Pressform (1) befestigt werden kann und die dafür konfiguriert ist, bei jedem Schritt das in die Pressform (1) gegossene Abdeckmaterial (X1, X2) und Aufschüttmaterial (Y1, Y2, Y3) schrittweise stoßweise zu verdichten,
einen Luftkompressor (300), der dafür konfiguriert ist, die Luftdruckpressvorrichtung (100) zu betätigen, und
einen Speicherplatz (400), der zur Aufbewahrung der Zusatzgeräte für den Mischer (200A, 200B), für die Pressform (1) und die Luftdruckpressvorrichtung (100) konfiguriert ist,
wobei die Mischer (200A, 200B) und der Luftkompressor (300) in einer rechten und linken Richtung an jeder Seite der Luftdruckpressvorrichtung (100) und benachbart zu ihr angeordnet sind, und wobei der Speicherplatz (400) in einer vorderseitigen und rückwärtigen Richtung zur Luftdruckpressvorrichtung (100) mit Arbeitsplatz (500) zwischen dem Speicherplatz (400) und der Luftdruckpressvorrichtung (100) angeordnet ist.

2. System zur Herstellung eines Prüflings nach Anspruch 1, **dadurch gekennzeichnet, dass** das System auf einer Ladefläche eines Lastkraftwagens derart angeordnet ist, dass die rechte und linke Richtung des Herstellungssystems der vorderseitigen und der rückseitigen Richtung des Lastkraftwagens entspricht.

3. Verfahren zur Herstellung eines Prüflings, der einen Zustand reproduziert, in welchem ein Aufschüttmaterial (Y1, Y2, Y3), das anorganischen Abfall enthält, deponiert wird, **dadurch gekennzeichnet, dass** es umfasst:
einen ersten Schritt des Gießens eines Abdeckmaterials (X1, X2) auf den Boden einer zylindrischen Pressform (1) mit flachem Boden und des Komprimierens des zu verdichtenden Abdeckmaterials (X1, X2), wodurch eine untere Abdeckschicht des verdichteten Abdeckmaterials (X1, X2) ausgebildet wird,
einen zweiten Schritt des Gießens des Aufschüttmaterials (Y1, Y2, Y3) auf das Abdeckmaterial (X1, X2), das in dem ersten Schritt die untere Abdeckschicht ausbildet, und des Komprimierens des Aufschüttmaterials (Y1, Y2, Y3), wobei das Aufschüttmaterial (Y1, Y2, Y3) in einem Verfestigungszustand ausgebildet wird,
einen dritten Schritt des Gießens des Abdeckmaterials (X1, X2) auf das Auf schüttmaterial (Y1, Y2, Y3) in einem Verfestigungszustand, der mit dem zweiten Schritt ausgebildet wurde, und des Komprimierens des zu verdichtenden Abdeckmaterials (X1, X2), wodurch eine obere Abdeckschicht des verdichteten Abdeckmaterials (X1, X2) ausgebildet wird, und
einen vierten Schritt des Fixierens des Oberteils des Abdeckmaterials (X1, X2), das in dem dritten Schritt die obere Abdeckschicht ausbildet, durch einen Deckel (5), wobei der Deckel (5) das Abdeckmaterial (X1, X2) selbst dann durch Druck fixiert, wenn sich das Aufschüttmaterial (Y1, Y2, Y3) ausdehnt.

4. Verfahren zur Herstellung eines Prüflings nach Anspruch 3, **dadurch gekennzeichnet, dass** es Vorbehandlungsschritte zum Ausbringen eines Kunstharzmaterials (2, 3, 4) zur Delaminierung auf einer Innenfläche der Pressform (1) vor dem ersten Schritt umfasst.

5. Verfahren zur Herstellung eines Prüflings nach Anspruch 3 oder 4, **dadurch gekennzeichnet, dass** es einen Nachbehandlungsschritt des Aushärtens des Prüflings über einen gewissen Zeitraum umfasst, während das Abdeckmaterial (X1, X2), das im vierten Schritt die obere Abschlussschicht ausbildet, mit dem Deckel (5) fixiert wird, und wobei dann der Deckel (5) sowie die Pressform (1) entfernt werden.

## Revendications

1. Système pour fabriquer une éprouvette qui reproduit un état dans lequel un matériau de décharge (Y1, Y2, Y3) qui contient des déchets non organiques est utilisé comme remblai, comprenant :
- des malaxeurs (200A, 200B) pour produire le matériau de remblaiement (Y1, Y2, Y3) et un matériau de couverture (X1, X2) qui couvre le matériau de remblaiement (Y1, Y2, Y3) respectivement en le mélangeant,
- un moule cylindrique à fond plat (1),
système **caractérisé en ce qu'**il comprend en outre :
- un couvercle amovible (5) pour le moule, configuré pour bloquer en pression le matériau de couverture (X1, X2) même si le matériau de remblaiement (Y1, Y2, Y3) se dilate,
- un dispositif à air comprimé (100) ayant une embase (110) à laquelle on peut fixer le moule (1) et qui est configurée pour comprimer par choc à chaque étape, le matériau de couverture (X1, X2) et le matériau de remblaiement (Y1, Y2, Y3) déversés dans le moule (1) d'une manière pas à pas,
- un compresseur pneumatique (300) pour actionner le dispositif à air comprimé (100), et
- un espace de stockage (400) pour loger les accessoires du mélangeur (200A, 200B), du moule (1) et du dispositif à air comprimé (100),
- les malaxeurs (200A, 200B) et le compresseur pneumatique (300) étant situés dans la direction à droite et dans la direction à gauche de chaque côté et de façon adjacente au dispositif à air comprimé (100) et l'espace de stockage (400) est disposé en avant et en arrière du dispositif à air comprimé (100) avec un espace de travail (500) entre l'espace de stockage (400) et le dispositif à air comprimé (100).

2. Système de production d'une éprouvette selon la revendication 1,
**caractérisé en ce qu'**
il est installé sur la plateforme d'un véhicule utilitaire de façon que la direction à droite et la direction à gauche du système de production correspondent à la direction avant et à la direction arrière du véhicule de transport.

3. Procédé de fabrication d'une éprouvette qui reproduit l'état dans lequel un matériau de remblaiement (Y1, Y2, Y3) qui contient un déchet non organique est utilisé comme remblai,
procédé **caractérisé en ce qu'**il comprend :
- une première étape consistant à déverser un matériau de couverture (X1, X2) au fond du moule cylindrique à fond plat (1) et à comprimer le matériau de couverture (X1, X2) à compacter pour produire une couche inférieure de recouvrement du matériau de couverture compacté (X1, X2),
- une seconde étape de déversement du matériau de remblaiement (Y1, Y2, Y3) sur le matériau de couverture (X1, X2) pour former la couche inférieure de revêtement dans la première étape et comprimer le matériau de remblaiement (Y1, Y2, Y3) et reproduire le matériau de remblaiement (Y1, Y2, Y3) à l'état consolidé,
- une troisième étape de déversement du matériau de couverture (X1, X2) sur le matériau de remblai (X1, Y2, Y3) dans un état consolidé, avec la seconde étape et à comprimer le matériau de couverture (X1, X2) pour être compacté et reproduire une couche de recouvrement supérieure du matériau de couverture compacté (X1, X2), et
- une quatrième étape de fixation du dessus du matériau de couverture (X1, X2) reproduisant la couche enveloppe supérieure dans la troisième étape par un couvercle (5), ce couvercle (5) bloquant la pression du matériau de couverture (X1, X2) même si le matériau de remblai (Y1, Y2, Y3) se dilate.

4. Procédé de production d'une éprouvette selon la revendication 3,
**caractérisé en ce qu'**
on effectue des étapes de prétraitement en plaçant une résine (2, 3, 4) pour l'exfoliation de la surface intérieure du moule (1) avant la première étape.

5. Procédé de production d'une éprouvette selon la revendication 3 ou 4,
**caractérisé en ce qu'**
on effectue une étape de prétraitement de cuisson de l'éprouvette pour une certaine période pendant laquelle le matériau de couverture (X1, X2) produit une couche de couverture supérieure dans la quatrième étape et se fixe au couvercle (5), puis on enlève le couvercle (5) et le moule (1).
